# EUROPEAN PATENT APPLICATION

(11) **EP 2 583 623 A1**
(43) Date of publication of application: **24.04.2013**
(21) Application number: 11798024.3
(22) Date of filing: 15.06.2011
(51) Int. Cl.: A61B 5/11, A61B 5/00

(54) **LIVING ORGANISM INFORMATION DETECTION SYSTEM**

(30) Priority: 21.06.2010 JP 2010140186
(71) Applicant: Aisin Seiki Kabushiki Kaisha, Aichi 448-8650 (JP)
(72) Inventor: IMAMURA, Ayako, Kariya-shi Aichi 448-8650 (JP); OHIRA, Tetsuya, Kariya-shi Aichi 448-8650 (JP)
(74) Representative: Winter, Brandl, Fürniss, Hübner, Röss, Kaiser, Polte - Partnerschaft
(86) International application number: PCT/JP2011/063660
(87) International publication number: WO 2011/162138

(57) **Abstract**

For providing a living organism information detection system that is capable of extracting, with high precision and inexpensive structure, living organism information including a heartbeat signal, the living organism information system is configured to include a vibration detection unit acquiring simultaneously vibrations of multiple pieces of living organism information by at least one sensor and outputting a vibration signal; an amplification unit having a first filter and a second filter with a longer time constant than the first filter, the first and second filters being inputted with vibrations of two pieces of living organism information among the multiple pieces of living organism information, the amplification unit amplifying the acquired vibration signal so as to be in inverse proportion to the output signal of the first filter or the output signal of the second filter, whichever is smaller, the amplification unit outputting the amplified vibration signal; and a discrimination unit discriminating between the two pieces of living organism information by comparing the amplitude of the amplified vibration signal and a threshold.

## Description

### TECHNICAL FIELD

The invention relates to a living organism information detection system wherein living organism information of a human body supported on a human body support structure, examples of which are a bed and a vehicle seat, can be inexpensively and accurately collected.

### BACKGROUND ART

Conventionally, system designs have been developed to make sleep comfortable, wherein motions of a human body, which is an example of living organism information, are measured by a measuring apparatus, such as Actigraph, to determine a sleeping stage (state) to provide a better sleeping environment. In such an attempt, a living organism information measuring apparatus of unconstrained type was developed. The measuring apparatus is characterized by using a piezoelectric element to alleviate any physical burden on an examinee when information of the body motions is measured. According to the conventional apparatus disclosed in the Patent Document 1, for example, living organism information, such as heartbeats and body motions, are detected by a sensor in which the piezoelectric sensor is used (hereinafter, called piezoelectric sensor) and data thereby acquired is transmitted through two filters respectively adapted to specific frequency bands of the heartbeats and body motions (heartbeat filter and body motion filter) to extract frequency components thereof. Then, signals of the extracted frequency components are independently amplified by different amplifier circuits, and the number of heartbeats and the number of body motions are thereafter counted by different counter circuits.

### PRIOR ART DOCUMENT

### PATENT DOCUMENT

Patent Document 1: JP04-15038

### SUMMARY OF THE INVENTION

### PROBLEMS TO BE SOLVED BY THE INVENTION

According to the conventional apparatus disclosed in the Patent Document 1 provided with different filters and amplifier circuits to detect the different living organism information, a complex logic employed therein increases a computing load when a program is run, resulting in cost increase.

Another problem is a gain difference generated between the signals to be amplified by the amplifier circuits depending on a degree of contact between the piezoelectric sensor and the human body. If trying to assess the living organism information based on a fixed threshold alone to reduce the computing load, the signals need to be separately amplified in consideration of the gain difference. This actually increases the computing load rather than reducing it, resulting in cost increase.

According to the apparatus, the different pieces of living organism information are detected from the filters of different frequency bands. This possibly leads to a time lag between time axes of detection results respectively acquired because of different time constants of the filters. For example, there is inevitably a difference between a length of time during which a body motion once started comes to a halt and a length of time during which heartbeats temporarily exceeding an upper-limit threshold and not determined as heartbeat detectable are equal to or below the upper-limit threshold again and determined as heartbeat detectable. This makes it difficult to determine whether the heartbeats were undetectable simply because of a body motion or a particular condition in which the heartbeats were truly undetectable. As a result, a part of the heartbeat information may be left out.

The invention was accomplished to solve these conventional technical problems. The invention provides a living organism information detection system wherein living organism information, such as heartbeat signals, can be extracted inexpensively and accurately.

### MEASURES FOR SOLVING THE PROBLEMS

In order to solve the above described problems, a living organism information detection system according to Claim 1 comprises a vibration detection unit acquiring simultaneously vibrations of multiple pieces of living organism information that have different frequencies by at least one sensor that is provided on a human body support structure and outputting a vibration signal; an amplification unit including a first filter and a second filter having a longer time constant than the first filter that are connected in parallel to the vibration detection unit, the first and second filters being inputted with vibrations of two pieces of living organism information, respectively, among the multiple pieces of living organism information that are of different amplitudes, the amplification unit performing an amplification of the vibration signal acquired by the vibration detection unit and outputting an amplified vibration signal, the amplification being established so as to be in reverse proportion to an output signal of the first filter or an output signal of the second filter, whichever is smaller; and a discrimination unit inputted with the amplified vibration signal and discriminating between the two pieces of living organism information.

In order to solve the above described problems, a living organism information detection system according to Claim 2 resides in that in Claim 1 further comprising a filtering unit being inputted with the amplified vibration signal, filtering the vibrations of the two pieces of living organism information, and outputting a filtered vibration signal.

In order to solve the above described problems, a living organism information detection system according to Claim 3 resides in that in Claim 1 or 2, the two pieces of living organism information are at least one of a set of heartbeats and body motions and a set of breaths and body motions that are among the multiple pieces of living organism information.

In order to solve the above described problems, a living organism information detection system according to Claim 4 resides in that in Claim 1 or 2, the human body support structure is a bedding tool, and the two pieces of living organism information are heartbeats and body motions.

In order to solve the above described problems, a living organism information detection system according to Claim 5 resides in that in Claim 1 or 2, the human body support structure is a vehicle seat, and the two pieces of living organism information are breaths and body motions.

In order to solve the above described problems, a living organism information detection system according to Claim 6 resides in that in Claim 1 to 5, a piezoelectric element constitutes the sensor.

### EFFECTS OF THE INVENTION

According to the living organism information detection system in Claim 1, wherein the signal acquired by the sensor and containing two pieces of living organism information respectively having different vibrational amplitudes and frequencies is inputted to and processed in the first filter and the second filter having a time constant longer than that of the first filter and then outputted. When the second filter having a time constant longer than that of the first filter is used as well as the first filter, a long-frequency signal of the living organism information can be extracted as a signal having suitable small output value. Of the output signals of the first and second filters, one of the output signals having a smaller output value than the other is extracted, and the vibration signal acquired by the sensor is amplified in reverse proportion to the output signal value. Then, the amplified vibration signal taking into consideration a gain difference of the sensor and containing the two pieces of normalized living organism information is favorably acquired. The two pieces of living organism information are discriminated from each other based on the normalized amplified vibration signal. Therefore, just a type of threshold value needs to be prepared in advance as a basic value for discrimination. This technical feature lessens a computing load, contributing to a simplified system configuration that can be provided at low cost.

Additionally, the signal is transmitted through the first and second filters with the two pieces of living organism information both included therein, no time lag is generated between the two pieces of living organism information, therefore, there is no information left out.

According to the living organism information detection system in Claim 2, wherein the filtering unit makes the amplified vibration signal including the two pieces of living organism information transmit through one filter to smooth the two pieces of living organism information before the discriminating process. This technical feature helps to accurately perform the comparison to the threshold value, and also eliminates the risk of any left-out information because of no time lag between the two pieces of living organism information.

According to the living organism information detection system in Claim 3, wherein the heartbeats and body motions or the breaths and body motions respectively having near frequencies are processed to facilitate the comparison. As a result, the living organism information can be very accurately extracted.

According to the living organism information detection system in claim 4, wherein the vibration data emitted from a subject lying on the bedding tool is acquired to detect the heartbeats and body motions which are examples of the living organism information. This is a technical feature suitable for confirming the physical condition of a subject that should be regularly checked (including subjects in illness and in good health).

According to the living organism information detection system in claim 5, wherein the vibration data emitted from a passenger seated on the vehicle seat is acquired to detect the breaths and body motions which are examples of the living organism information, the passenger's physical condition can be known suitably and accurately.

According to the living organism information detection system in claim 6, wherein the piezoelectric element constitutes the sensor. This technical feature exerts the following advantages; the vitiation signals in a broad range of frequencies are acquired, responsiveness, accuracy, and cost reduction can be served well, and the sensor that can be provided in a small thickness is hardly felt like a foreign object by a subject lying on the bedding tool or seated on the vehicle seat when placed under him/her.

### BRIEF DESCRIPTION OF THE DRAWINGS

Figs. 1 are conceptual drawings of a living organism information detection system 1 according to a first embodiment.
Fig. 2 is an illustration of vibration signals outputted from piezoelectric sensors according to the first and second embodiments.
Fig. 3 is a graphical illustration of the vibration signals after being transmitted through first and second filters constituting an amplification unit according to the first and second embodiments.
Fig. 4 is a control flow chart according to the first and second embodiments.
Figs. 5 are graphical illustrations to describe about a discrimination unit after filtered according to the first embodiment.
Fig. 6 is a conceptual drawing of a living organism information detection system 2 according the second embodiment.
Figs. 7 are graphical illustrations to describe about a discrimination unit after filtered according to the second embodiment.

### EMBODIMENTS FOR PRACTICING THE INVENTION

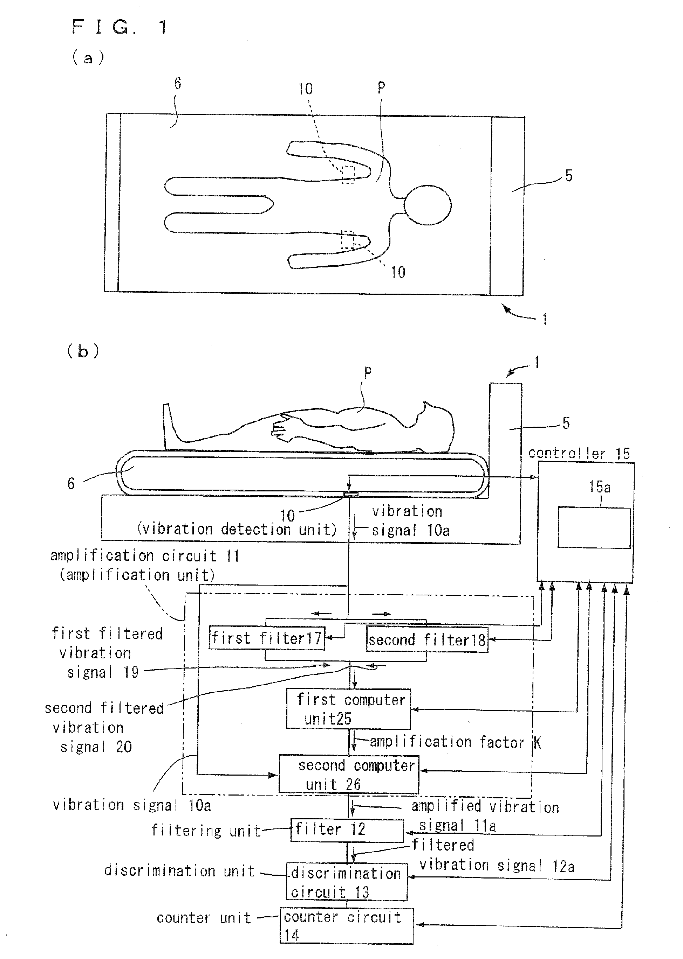

Hereinafter, a first embodiment of a living organism information detection system according to the invention is described in detail referring to the accompanied drawings. As illustrated in Figs. 1 (a) and (b), a living organism information detection system 1 includes two piezoelectric sensors 10 for detecting vibrations, each serving as a vibration detection unit provided on a bed body 5 (or mattress 6) which is a bedding tool comparable to the human body support structure according to the invention and an amplification circuit 11 serving as an amplification unit for amplifying vibration signals 10a outputted from the piezoelectric sensors 10 to a normalized value described later. The living organism information detection system 1 further includes: a filter 12 serving as a filtering unit for smoothing amplified vibration signals 11a amplified by the amplification circuit 11; a discrimination circuit 13 serving as a discrimination unit for comparing a filter-transmitted vibration signal 12a outputted from the filter 12 to predefined threshold values S1 and S2 described later to discriminate a heartbeat signal H and a body motion signal M from each other; a counter circuit 14 serving as a counter unit for counting the number of heartbeats of the heartbeat signal H and the number of body motions; and a controller 15.

The present embodiment provides two piezoelectric sensors 10, however, structural and operational characteristics of these piezoelectric sensors 10 are identical. In most parts of the description given below, therefore, only one of the piezoelectric sensors 10 is described. Ultimately, it is decided that data of only one of the piezoelectric sensors 10 is used, and a method of selecting the data to be used will be described later.

The piezoelectric sensor 10 is formed in the shape of a planar sheet having flexibility. A piezoelectric body (piezoelectric element) constitutes the piezoelectric sensor 10, examples of which are piezoelectric polymers and piezoelectric ceramics. A specific example is a film or a sheet made from polyvinylidene fluoride (PVDF). The piezoelectric polymers are characterized by broad frequencies and have better flexibility, shock resistance, water resistance, resistance to high voltages, and chemical stability than the piezoelectric ceramics. Other examples of the vibration detection unit are a capacitance sensor, a strain gauge, and a magnetic sensor.

As illustrated in Figs. 1(a) and (b), the two piezoelectric sensors 10 are interposed between a lower surface of the mattress 6 and an upper surface of the bed body 5 at positions in the direction of gravity below the chest of a living body P lying on the bed body 5. The two piezoelectric sensors 10 are spaced from each other by a given distance from the center of the bed body 5 in a width direction thereof toward outer sides of the bed body 5 in the width direction (refer to Fig. 1(a)). Because of the location of the piezoelectric sensors 10, when the living body P lying on the mattress 6 (bed body 5) moves toward either side of the mattress 6 (bed body 5) in the width direction, at least one of the two piezoelectric sensors 10 surely detects multiple pieces of living organism information of the living body P, specifically, information of heartbeats, breaths, and body motions such as rolling over.

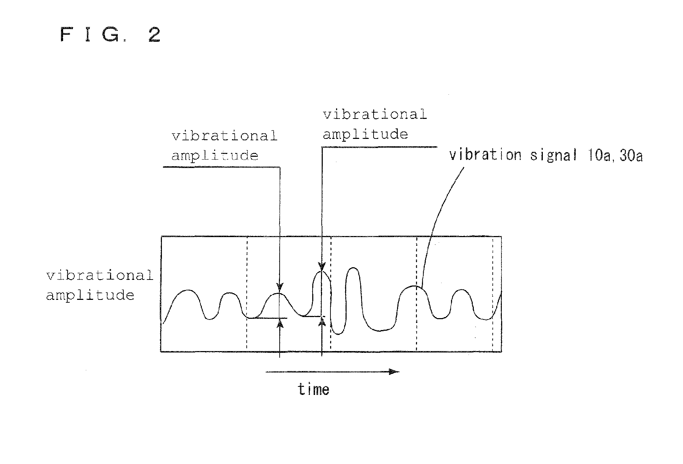

The piezoelectric sensors10 detect simultaneously any changes (accelerations) occurring in vibrations of multiple pieces of living organism information acquired from the living body P (heartbeats, breaths, and body motions) and outputs the vibration signals 10a (refer to Figs. 1 and 2). The number of the piezoelectric sensors 10 is not necessarily limited to two, and more than two piezoelectric sensors 10 (for example, six) may be provided at given positions. The location of the piezoelectric sensors 10 is not necessarily limited to the lower surface of the mattress 6 but may be an upper surface thereof.

The amplification circuit 11 illustrated in Fig. 1, which is an example of the amplification unit, is described below. The amplification circuit 11 is provided to amplify the vibration signal 10a outputted from the piezoelectric sensor 10 to the normalized value described earlier. The normalization is to adjust a gain of the vibration signal 10a acquired and outputted by the piezoelectric sensor 10 to the normalized value. Similarly to any conventional sensors, the gain of the vibration signal outputted from the piezoelectric sensor 10 is variable depending on, for example, location of the piezoelectric sensor 10 relative to the living body P and pressing of the piezoelectric sensors 10 to the living body P. To amplify all of the vibrations signals 10a outputted from the piezoelectric sensors 10 by an equal amplification factor and discriminate the different pieces of living organism information based on the amplified signals thus acquired, it is necessary to compute different threshold values adapted to the respective sensor gains which differ depending on measuring requirements. This, however, increases a computing load, imposing a heavier operational burden on the controller 15.

To reduce the computing load, the invention focused on different signal values of the vibration signals 10a outputted from the piezoelectric sensors 10 because of different gains. The amplification factors suitable for the signal values of the vibration signals 10a, in other words, gain values, are respectively calculated based on the signal values, and the vibration signals 10a are amplified by the calculated amplification factors to be normalized. Then, the normalized amplified output values are compared to the common threshold value S1 and S2 to discriminate all of the vibration signals 10a outputted from the piezoelectric sensors 10. To serve the purpose, the amplification circuit 11 derives a predefined amplification factor K described later and amplifies the vibration signal 10a outputted from the piezoelectric sensor 10 by the amplification factor K to calculate the normalized value.

The amplification circuit 11 is connected to the piezoelectric sensors 10. The amplification circuit 11 is provided with a first filter 17 having time constants suitably set therein for vibration frequencies of the heartbeat signal H and the body motion signal M, a second filter 18 having time constants longer than those of the first filter 17 set therein, a first computer unit 25, and a second computer unit 26. According to the first embodiment, the vibration signal 10a mixedly including the heartbeat signal H and the body motion signal M of near frequencies but different amplitude magnitudes, which are at least two pieces of living organism information included in multiple pieces of living organism information, is inputted to each of the first filter 17 and the second filter 18.

The first filter 17 and the second filter 18 are connected in parallel to the piezoelectric sensors 10. The first filter 17 and the second filter 18 are further connected in parallel to the first computer unit 25 provided in a subsequent processing section, and the second computer unit 26 is connected in series to the first computer unit 25.

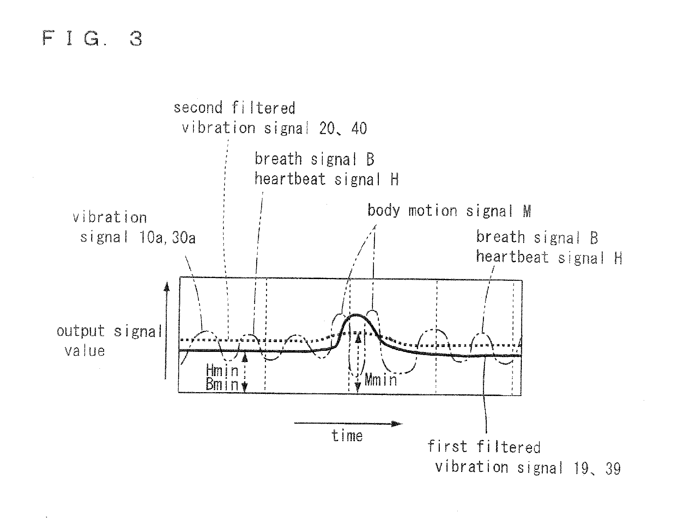

The amplification factor K used to amplify and normalize the vibration signal 10a is described below. To derive the amplification factor K, the vibration signals 10a are transmitted through the first filter 17 and the second filter 18 to output first and second filtered vibration signals 19 and 20 which are output signals of the respective vibration signals (refer to Figs .1 and 3). The first filter 17 has relatively short time constants set therein in accordance with the frequency of the heartbeat signal H. Then, as illustrated with a solid line in Fig. 3, the heartbeat signal H of the first filtered vibration signal 19 transmitted through the first filer 17 shows a substantially average value of vibrational amplitudes of heartbeats which is a relatively small output signal value. The body motion signal M of the first filtered vibration signal 19 shows a relatively large output signal value under the influence of the magnitude of body motions in the vibration signal 10a because of relatively short time constants of the first filter 17.

The second filter 18 has relatively long time constants set therein than the first filter 17 in accordance with the frequency of the body motion signal M. Then, as illustrated with a broken line in Fig. 3, the body motion signal M of the second filtered vibration signal 20 transmitted through the second filer 18 shows a relatively small output signal value, which is suitable for amplification as described later, under the influence of the heartbeat signal H of the vibration signal 10a. Because of relatively long time constants of the second filter 18, a part of the second filtered vibration signal 20 indicating heartbeats shows an output signal value slightly larger than a part of the first filtered vibration signal 19 indicating heartbeats.

As illustrated in Fig. 3, the first and second filtered vibration signals 19 and 20 are overlapped on an equal time axis and compared to each other to extract smaller output values H min and M min illustrated with arrows in Fig. 3. Then, one of H min and M min even smaller than the other is selected to calculate an inverse number 1/Hmin or 1/Mmin and thereby calculate the amplification factor K of the vibration signals 10a (first computer unit 25).

When the second filter 18 having relatively long time constants are used as well as the first filter 17 having short time constants, a small output value of the long-frequency body motion signal M, which is suitable for the amplification, can be extracted and used as a basic value to obtain the appropriate amplification factor K.

The output signal values H min and M min of the first and second filtered vibration signals 19 and 20 including the heartbeat signal H and the body motion signal M, which are used to calculate the inverse number, are desirably equal to or smaller than 1. Accordingly, the amplification factor K, which is the inverse number of one of the output signal values H min and M min smaller than the other, exceeds the value of 1, ensuring the amplifying process. In the case where the output signal values H min and M min of the heartbeat signal H and the body motion signal M are larger than 1 and the inverse numbers thereof are smaller than 1, in addition to the calculation of the inverse numbers, the inverse numbers are multiplied by an arbitrary multiplying factor to obtain the amplification factor K exceeding 1.

The second computer unit 26 multiplies the vibration signal 10a initially acquired by the piezoelectric sensor 10 by the calculated amplification factor K and outputs the normalized amplified vibration signal 11a.

Thus, the amplification factor K takes the inverse number of one of the output signal values H min and M min of the first and second filtered vibration signals 19 and 20 smaller than the other. Therefore, the amplification factor K has a smaller value as the output signal values H min and M min are larger. On the other hand, the amplification factor K has a larger value as one of the output signal values H min and M min smaller than the other is further smaller. Thus, the output signal values H min , M min and the amplification factor K are in reverse proportion to each other.

As described so far, the vibration signal 10a including the heartbeat signal H and the body motion signal M acquired and outputted by the piezoelectric sensor 10 is amplified by the amplification factor K in reverse proportion to one of the output signal values H min and M min smaller than the other in the heartbeat signal H and the body motion signal M of the first and second filtered vibration signals 19 and 20 irrespective of any gains of the piezoelectric sensor10. The vibration signal 10a is thus amplified and outputted as the normalized amplified vibration signal 11a.

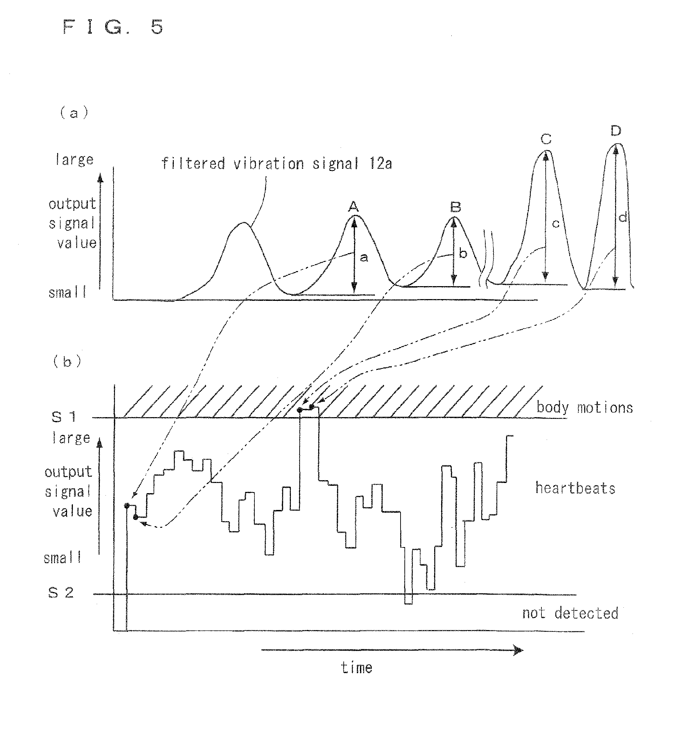

The filter 12, which is an example of the filtering unit, is provided to smooth the amplified vibration signal 11a. An example of the smoothing is averaging, wherein the amplified vibration signal 11a finely vibrating is averaged to acquire smoothed data. To smooth the amplified vibration signal 11a, the filter 12 has time constants which cover all of frequency bands of the heartbeat signal H and the body motion signal M which are two examples of the living organism information. When the amplified vibration signal 11a including the heartbeat signal H and the body motion signal M is transmitted through the filter 12, the smoothed filtered vibration signal 12a illustrated in Figs. 1 and 5(a), is outputted.

To discriminate the heartbeat signal H and the body motion signal M which are two examples of the living organism information, the discrimination circuit 13 as the discrimination unit converts the filtered vibration signal 12a illustrated in Fig. 5(a) in the form of a graphical illustration of Fig. 5 (b). Height dimensions a, b, c, and d of points A, B, C, and D illustrated in Fig. 5 (a) are sequentially measured and plotted in the graph of Fig. 5(b). Then, the heartbeat threshold values S1 and S2 previously set, which were described earlier, are superimposed on the graph of Fig. 5(b), and the respective plotted data are compared to the threshold values S1 and S2 to discriminate the heartbeat signal H and the body motion signal M, a signal exceeding the heartbeats, from each other.

The threshold values S1 and S2 are calculated in advance and stored in a storage 15a (refer to Fig. 1) of the controller 15. The threshold values S1 and S2 are set to suitable values based on a large number of heartbeat data previously measured. Of the outputs of the filtered vibration signal 12a, outputs indicated by the points A and B between the threshold values S1 and S2 are the heartbeat signals H. In the case where the filtered vibration signal 12a illustrated in Fig. 5(a) has such data that is indicated by the points C and D, the outputs illustrated in Fig. 5(b) exceeding the threshold value S1 are included in the area of body motions, therefore, discriminated as data of body motions. The outputs smaller than the threshold value S2 are determined as no heartbeat signal H being detected and accordingly processed.

Thus, the heartbeats and the body motions having near frequency bands are acquired and transmitted at the same time through the first filter 17 and the second filter 18, and the amplification factor K is calculated based on the minimal value of the first and second filtered vibration signal 19 and 20. Then, the heartbeats and the body motions are amplified by the calculated amplification factor K and smoothed at the same time by one filter 12. In this manner, any time lag is not generated between the two pieces of living organism information, and the living organism information can be very accurately extracted.

The counter circuit 14 counts the number of heartbeats of the heartbeat signal H and the number of body motions of the body motion signal M. To count the number of heartbeats, the counter circuit 14 counts amplitude peaks of the filtered vibration signal 12a. According to the first embodiment, the heartbeats of the heartbeat signal H per unit of time of the two piezoelectric sensors 10 are compared to each other, and one of the data having a higher detection rate for the heartbeat signal H is selected. The same goes for more than two piezoelectric sensors 10. Whendetermined as body motions by the discrimination circuit 13, an interval of an uninterrupted body motion is counted as a body motion and the counted intervals in total are used as the number of body motions.

As illustrated in Fig. 1, the controller 15 is connected to the piezoelectric sensors 10, amplification circuit 11 (first filter 17, second filter 18, first computer unit 25, and second computer unit 26), filter 12, discrimination circuit 13, and counter circuit 14. The controller 15 transmits and receives data to and from the piezoelectric sensors 10, the filter 12, and the circuits 11, 13, and 14, and controls these structural elements.

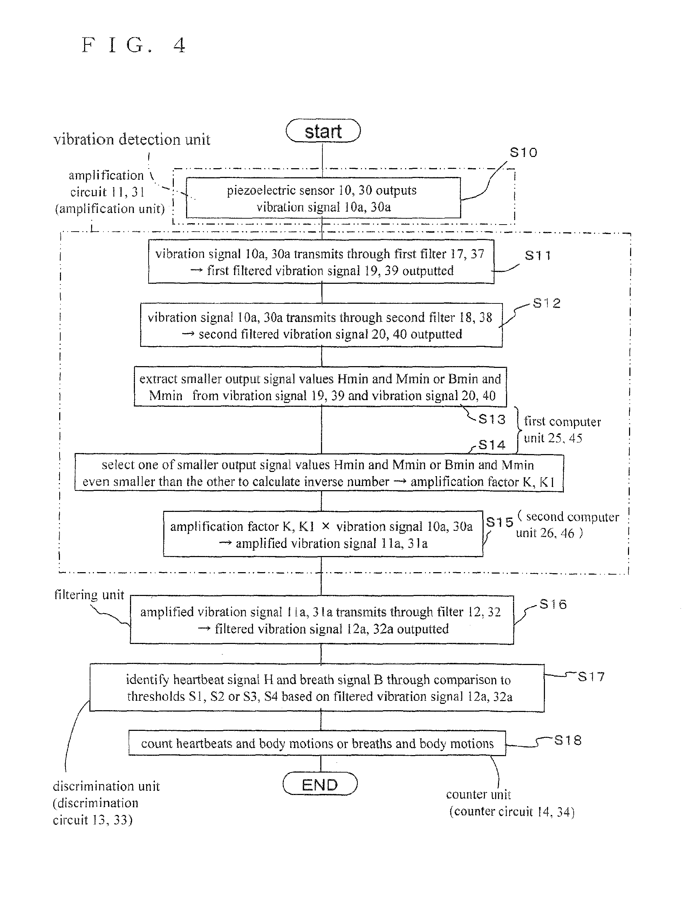

Referring to a flow chart illustrated in Fig. 4, operational advantages according to the first embodiment are described. First, a detection start switch not illustrated in the drawings is turned on to activate the piezoelectric sensors 10 which are as vibration detection units provided on the bed body 5.

In Step S10, the piezoelectric sensor 10 (vibration detection units) detects vibrations generated by the living body P (for example, heartbeats, body motions) and outputs the detected vibrations in the form of the vibration signal 10a.

In Step S11, the vibration signal 10a is transmitted through the first filter 17, and the first filtered vibration signal 19 is outputted (refer to Fig. 3). The first filter 17 has short time constants set therein. Therefore, of the signals included in the outputted first filtered vibration signal 19, the heartbeat signal H has a small output signal value substantially equal to the average value, while the body motion signal M has a large output signal value.

In Step S12, the vibration signal 10a is transmitted through the second filter 18, and the second filtered vibration signal 20 is outputted (refer to Fig. 3). The second filter 18 has long time constants set therein. Therefore, the signal value of a part of the outputted second filtered vibration signal 20 indicating heartbeats has an output signal value slightly larger than the signal value of a part of the first filtered vibration signal 19 indicating heartbeats. On the other hand, the signal value of a part of the second filtered vibration signal 20 indicating body motions has an output signal value smaller than the signal value of a part of the first filtered vibration signal 19 indicating body motions (refer to Fig. 3). The first filter 17 and the second filter 18 are illustrated in this order in the respective flows of Step S11 and Step S12. In an actual operation, however, the constantly operating first and second filters 17 and 18 concurrently execute these processing steps.

In Step S13, of the smaller output signal values of the first filtered vibration signal 19 and the second filtered vibration signal 20, the value H min of the first filtered vibration signal 19 is extracted from the heartbeat indicating part, while the value M min of the second filtered vibration signal 20 is extracted from the body-motion indicating part.

In Step S14, one of the output signal values H min and M min even smaller than the other is selected to obtain the inverse number and thereby calculate the amplification factor K (Steps 13 and S14 are the processing steps by the first computer unit 25).

In Step S15 (by the second computer unit 26), the vibration signal 10a is multiplied by the amplification factor K to output the normalized amplified vibration signal 11a.

In Step S16, the amplified vibration signal 11a is transmitted through the second filter 12 (filtering unit) to be smoothed, and the filtered vibration signal 12a is outputted.

In Step S17, the signal of Fig. 5 (b) processed based on the filtered vibration signal 12a by the discrimination circuit 13 (discrimination unit) is compared to the threshold values S1 and S2 stored in the storage 15a of the controller 15 to discriminate the heartbeat signal H and the body motion signal M from each other.

In Step S18, peak values of the filtered vibration signal 12a determined as the heartbeat signal H are counted by the counter circuit 14 (counter unit) and written as data in the storage 15a of the controller 15. Further, peak values of the filtered vibration signal 12a determined as the body motion signal M are counted by the counter circuit 14. As a result of these processing steps, the heartbeat signal H can be very accurately identified (extracted) and counted. The heartbeat signals H thus counted are useful for a variety of health managements.

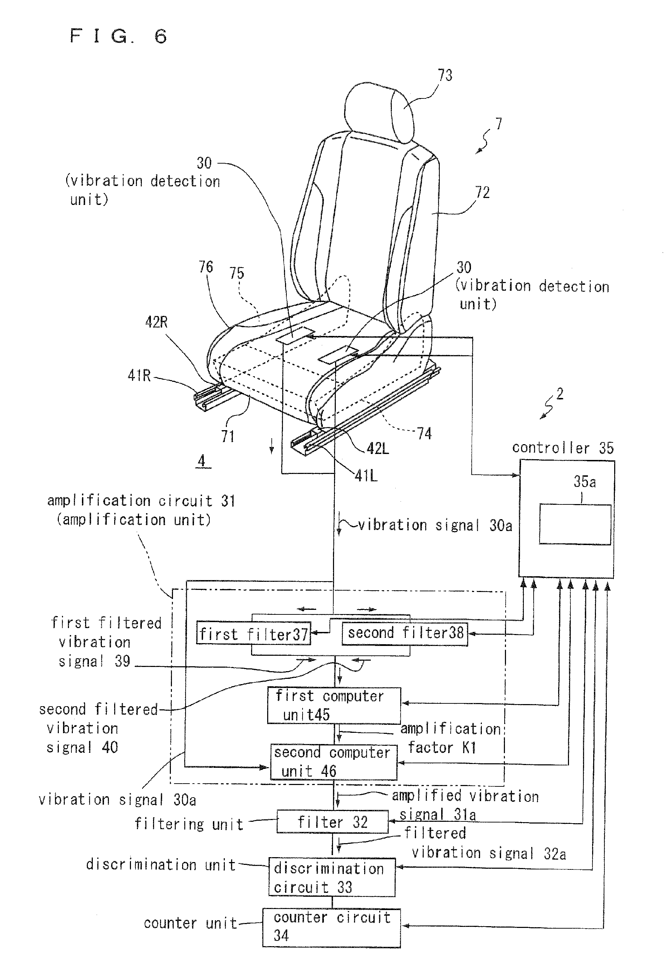

Hereinafter, a second embodiment of the living organism information detection system according to the invention is described referring to the accompanied drawings. As illustrated in Fig. 6, a living organism information detection system 2 according to the second embodiment includes two piezoelectric sensors 30 (an example of the vibration detection unit according to the invention) for detecting vibrations provided on a seating surface of a vehicle seat 7 (an example of the human body support structure according to the invention), and an amplification circuit 31 (an example of the amplification unit according to the invention) for amplifying vibration signals 30a outputted from the piezoelectric sensors 30 to a normalized value. The living organism information detection system 2 further includes a filer 32 (an example of the filtering unit according to the invention) for smoothing amplified vibration signals 31a amplified by the amplification circuit 31, and a discrimination circuit 33 (an example of the discrimination unit according to the invention) for comparing filtered vibration signals 32a outputted from the filter 32 to predefined threshold values S3 and S4 described later to discriminate a breath signal B and a body motion signal M from each other. The living organism information detection system 2 further includes a counter circuit 34 (an example of the counter unit according to the invention) for counting the number of breaths of the breath signal B and the number of body motions of the body motion signal M and a controller 35. The controller 35 is connected to the piezoelectric sensors 30, amplification circuit 31 (first filter 37, second filter 38, first computer unit 45, and second computer unit 46), filter 32, discrimination circuit 33, and counter circuit 44. The controller 35 is connected to each of the first filter 37, second filter 38, first computer unit 45, and second computer unit 46 of the amplification circuit 31. The controller 35 transmits and receives data to and from the piezoelectric sensors 30, the filter 32, and the circuits 31, 33, and 34, and controls these structural elements.

The living organism information detection system 2 according to the second embodiment is different from the living organism information detection system 1 according to the first embodiment in that the bedding tool (bed body 5) provided as the human body support structure is replaced with the vehicle seat 7. Hereinafter, technical differences thereby generated are described, while description of any other similar features is omitted.

As illustrated in Fig. 6, the vehicle seat 7 has a seat cushion 71 where a passenger not illustrated in the drawing (living body P) is seated, and a seatback 72 where the passenger rest his/her back, the seatback 72 being attached to a rear end of the seat cushion 71 rotatably in a front - back direction. Further, a headrest 73 is attached to an upper end of the seatback 72 to support the passenger's head.

The seat cushion 71 includes a seat frame 74, a pad member 75 provided in an upper section of the seat frame 74, and a surficial member 76 provided to cover the surface of the pad member 75. To a lower surface of the seat frame 74 are attached a pair of upper rails 42L and 42R provided on left and right sides. The paired upper rails 42L and 42R are engaged with a pair of lower rails 41L and 41R secured to a vehicle floor 4 movably in the front - back direction.

The piezoelectric sensors 30, which are structurally and operationally configured similarly to the piezoelectric sensors 10 according to the first embodiment, arenotdescribedindetail. As illustrated in Fig. 6, the piezoelectric sensors 30 are provided on an upper surface of the surficial member 76 of the seat cushion 71 of the vehicle seat 7. The two piezoelectric sensors 30 are spaced from each other by a given distance from the center of the vehicle seat 7 in a width direction thereof toward outer sides of the vehicle seat 7. Because of the location of the piezoelectric sensors 30, when the passenger (living body P) seated on the seat cushion 71 of the vehicle seat 7 moves to either side in the width direction on the seat cushion 71, one of the piezoelectric sensors 30 surely detects information of vibrations caused by heartbeats, breaths, and body motions which are multiple pieces of living organism information of the passenger (living body P).

The piezoelectric sensors 30 detect simultaneously changes of the vibrations (accelerations) of multiple pieces of living organism information of the living body P (heartbeats, breaths, and body motions) and output vibration signals 30a including multiple pieces of living organism information (refer to Figs. 2 and 6). The number of the piezoelectric sensors 30 is not necessarily limited to two, and more than two piezoelectric sensors 30 (for example, six) may be provided at given positions. One piezoelectric sensor 30 maybe provided at given position. The piezoelectric sensors 30, though placed on the upper surface of the seat cushion 71, may be placed on a lower surface of the seat cushion 71, or may be placed between the surficial member 76 and the seat cushion 71.

The amplification circuit 31 is a device comparable to the amplification circuit 11 of the living organism information detection system 1 according to the first embodiment. The amplification circuit 31 is structurally and operationally configured similarly to the amplification circuit 11. The first filter 37, second filter 38, first computer unit 45, and second computer unit 46 of the amplification circuit 31 are devices comparable to the first filter 17, second filter 18, first computer unit 25, and second computer unit 26 of the living organism information detection system 1 and structurally and operationally configured similarly to these devices according to the first embodiment. The similarly configured structural elements are not described in detail. An amplification factor K1 is comparable to the amplification factor K according to the first embodiment.

Irrespective of the gains of the piezoelectric sensors 30, the vibration signals 30a including the breath signal B and the body motion signal M outputted by the piezoelectric sensors 30 (refer to Figs. 2 and 6) are amplified by the amplification factor K1 (= 1/Bmin or 1/Mmin) in reverse proportion to one of the output signal values B min and M min smaller than the other in the breath signal B and the body motion signal M of the first and second filtered vibration signals 39 and 40 illustrated in Fig. 3. The vibration signals 30a thus amplified and normalized are outputted as the amplified vibration signals 31a.

The filter 32 is a device comparable to the filter 12 according to the first embodiment. The filter 32 is structurally and operationally configured similarly to the filter 12. The discrimination circuit 33, counter circuit 34, and controller 35 are devices comparable to the discrimination circuit 13, counter circuit 14, and controller 15 according to the first embodiment and structurally and operationally configured similarly to these devices according to the first embodiment. A storage 35a of the controller 35 is comparable to the storage 15a of the controller 15 and structurally and operationally configured similarly thereto.

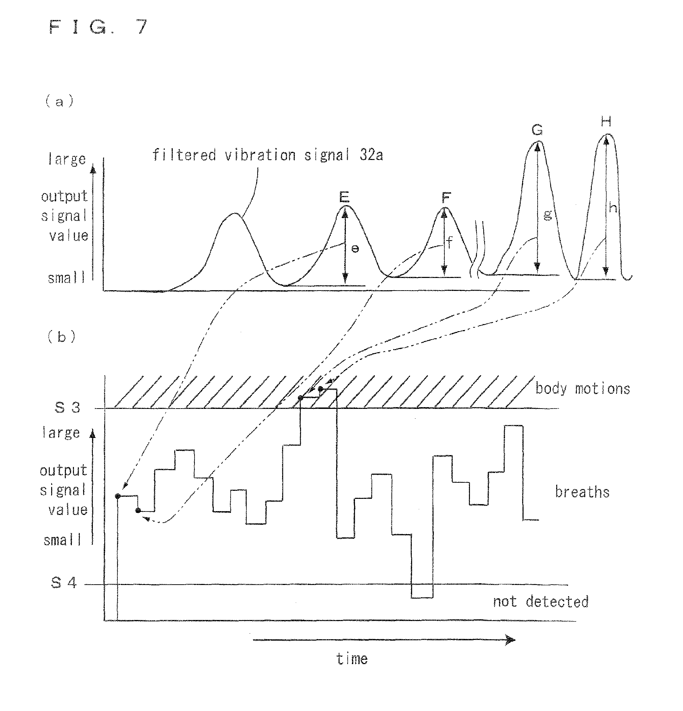

The amplified vibration signal 31a including the breath signal B and the body motion signal M is transmitted through the filter 32, and the filtered vibration signal 32a smoothed as illustrated in Figs. 6 and 7 is thereby outputted.

Height dimensions e, f, g, and h of points E, F, G, and H illustrated in Fig. 7(a) are sequentially measured and plotted by the discrimination circuit 33 in the graph of Fig. 7 (b). Then, the respective plotted data are compared to the threshold values S3 and S4 to discriminate the breath signal B and the body motion signal M, a signal exceeding the breaths, fromeachother. Of the outputs of the filtered vibration signal 32a, an output between the threshold values S3 and S4 is determined as the breath signal B, whereas the other output exceeding the threshold value S3 is determined as the body motion signal M. The output smaller than the threshold value S4 is determined as no breath signal B being detected and accordingly processed.

Similarly to the threshold values S1 and S2 of the living organism information detection system 1, the threshold values S3 and S4 are set to suitable values based on a large number of breath data previously measured and stored in the storage 35a of the controller 35.

Thus, the breaths and the body motions having near frequency bands are transmitted through the first filter 37 and the second filter 38 at the same time, and the amplification factor K1 is calculated by the first computer unit 45 based on the minimal value of the first and second filtered vibration signal 39 and 40. Then, these data are multiplied by the calculated amplification factor K1 and thereby amplified by the second computer unit 46, and then smoothed at once by one filter 32. In this manner, any time lag is not generated between the two pieces of living organism information, and the living organism information can be very accurately extracted.

Finally, the number of the breath signal B and the number of the body motion signal M are counted by the counter circuit 34. The counter circuit 34 counts the number of breaths of the breath signal B by counting the number of amplitude peaks of the filtered vibration signal 32a.

The operational advantages according to the second embodiment, which are similar to those of the first embodiment as illustrated in Steps S10 to S18 of the flow chart of Fig. 4, are not described. As a result of these processing steps, the breath signal B can be very accurately discriminated (extracted) from the body motion signal M and counted. The breath signals B thus counted are useful for a variety of health management.

According to the first and second embodiments, the living organism information to be collected are respectively two different combinations of signals; heartbeat signal H and body motion signal M, and breath signal B and body motion signal M. Instead of these combinations, the breath signal B and the body motion signal M may be collected as the pieces of living organism information on the bedding tool according to the first embodiment, while the heartbeat signal H and the body motion signal M may be collected as the pieces of living organism information on the vehicle seat 7 according to the second embodiment. Then, the breath signal B and the body motion signal M, and the heartbeat signal H and the body motion signal M, respectively are discriminated from each other based on the fixed (pair of) threshold values S3 and S4 and threshold values S1 and S2 alone prepared in advance, and the number of heartbeats, the number of breaths, and the number of body motions are respectively counted by the counter circuit.

As is clear from the description given so far, according to the living organism information detection system 1, 2 provided on the bedding tool, the vehicle seat which are examples of the human body support structure according to the first and second embodiment, the signal including two pieces of living organism information (heartbeats and body motion, or breaths and body motions)respectively having differentvibrational amplitudes and frequencies is acquired by the piezoelectric sensor 10, 30. Then, the acquired signal is inputted to, processed in, and outputted from the first filter 17, 37 and the second filter 18, 38 having longer time constants set therein than the first filter 17, 37. As a result, the body motion signal having a long frequency can be extracted as a suitably small output value. Then, the minimal value (H min or M min, or B min or M min) is selected from the first and second filtered output signals 19 and 20 (or 39 and 40) outputted from the first filter 17, the second filter 18 (or 37, 38) to calculate the inverse number and further calculate the amplification factor K, K1. The vibration signal 10a, 30a of eachpiezoelectric sensor 10, 30 is multiplied by the magnification factor K, K1 to acquire the amplified vibration signal 11a, 31a including the gain-considered two pieces of living organism information in which the heartbeats andbodymotions (orbreaths andbodymotions) are both normalized. Then, the heartbeats and body motions (or breaths or body motions) are discriminated from each other based on the normalized amplified vibration signal 11a, 31a as far as the fixed (pair of) threshold values S1 and S2 or S3 and S4 alone are prepared for discrimination in advance. This technical advantage lessens a computing load, contributing to a simplified system configuration that can be provided at low cost.

The vibration signal 10a,30a including the two pieces of living organism information having near frequencies, which are heartbeats and body motions, (or breaths and body motions) both is transmitted through the first filter 17, 37, second filter 18, 38, and filter 12, 32 which is a filtering unit for smoothing the amplified vibration signal 11a, 31a to discriminate the information from each other. In this manner, no time lag is generated between the two pieces of living organism information, therefore, there is no information left out.

The following advantages are exerted by the first and second embodiments wherein piezoelectric elements constitute the respective piezoelectric sensors 10 and 30; the vibration signals in a broad range of frequencies are acquired, responsiveness, accuracy, and cost reduction can be served well, and the sensor that can be provided in a small thickness is hardly felt like a foreign object by a subject lying on the bedding tool or seated on the vehicle seat when placed under him/her.

According to the first and second embodiments, the vibration detection unit is not necessarily limited to the piezoelectric sensor 10, 30andmaybe, for example, a load sensor or a vibration sensor. Thus, a wide selection range of devices are available, flexibly responding to the demand for cost reduction.

### INDUSTRIAL APPLICABILITY

The living organism information detection system according to the invention is suitably applied to beds of hospitals and nursing homes where living organism information of the living body P need to be acquired and vehicle seats where a passenger's living organism information should be acquired.

### DESCRIPTION OF SYMBOLS

1, 2... living organism information detection system, 5...human body support structure (bed body), 6... mattress, 7... human body support structure (vehicle seat), 10,30 ...vibration detection unit (piezoelectric sensor), 10a, 30a...vibration signal, 11, 31...amplification unit (amplification circuit), 11a, 31a...amplified vibration signal, 12,32...filtering unit (filter),12a, 32a...filtered vibration signal, 13, 33...discrimination unit (discrimination circuit), 14, 34...counter unit (counter circuit) 15, 35...controller, 17, 37...first filter, 18, 38... second filter, 19, 39... first filtered vibration signal, 20, 40... second filtered vibration signal, 25, 45... first computer unit, 26, 46... second computer unit, B...breath signal, H...heartbeat signal, M...body motion signal, P... living body, K, K1...amplification factor, S1, S2, S3, S4... threshold value.

## Claims

1. A living organism information detection system comprising:
a vibration detection unit acquiring simultaneously vibrations of multiple pieces of living organism information that have different frequencies by at least one sensor that is provided on a human body support structure and outputting a vibration signal;
an amplification unit including a first filter and a second filter having a longer time constant than the first filter that are connected in parallel to the vibration detection unit, the first and second filters being inputted with vibrations of two pieces of living organism information, respectively, among the multiple pieces of living organism information that are of different amplitudes,
the amplification unit performing an amplification of the vibration signal acquired by the vibration detection unit and outputting an amplified vibration signal, the amplification being established so as to be in reverse proportion to an output signal of the first filter or an output signal of the second filter, whichever is smaller; and
a discrimination unit inputted with the amplified vibration signal and discriminating between the two pieces of living organism information.

2. The living organism information detection system of Claim 1, further comprising a filtering unit being inputted with the amplified vibration signal, filtering the vibrations of the two pieces of living organism information, and outputting a filtered vibration signal.

3. The living organism information detection system of Claim 1 or 2, wherein the two pieces of living organism information are at least one of a set of heartbeats and body motions and a set of breaths and body motions that are among the multiple pieces of living organism information.

4. The living organism information detection system of Claim 1 or 2, wherein the human body support structure is a bedding tool, and the two pieces of living organism information are heartbeats and body motions.

5. The living organism information detection system of Claim 1 or 2, wherein the human body support structure is a vehicle seat, and the two pieces of living organism information are breaths and body motions.

6. The living organism information detection system of any one of claims 1 to 5, wherein a piezoelectric element constitutes the sensor.
